Europäisches Patentamt

European Patent Office

Office européen des brevets

(19)

(11) Publication number: **0 111 042**
**A1**

# (12) EUROPEAN PATENT APPLICATION

(21) Application number: 83100070.8

(22) Date of filing: 05.01.83

(51) Int. Cl.³: **G 01 N 33/52**
**G 01 N 33/54**

(30) Priority: 01.12.82 JP 210738/82

(43) Date of publication of application:
20.06.84 Bulletin 84/25

(84) Designated Contracting States:
BE CH DE FR GB IT LI SE

(71) Applicant: FUJI PHARMACEUTICAL IND. CO., LTD.
9-11, Shikahama, 1-chome
Adachi-ku Tokyo(JP)

(72) Inventor: Yokoi, Hideharu
39-3, Kamihoonji Kamiichimachi
Nakaniikawa-gun Toyama-ken(JP)

(72) Inventor: Shigeno, Yumiko
2-7-8, Yayoicho
Toyama-shi(JP)

(72) Inventor: Kitagawa, Keiko
177, Kuguminato
Shinminato-shi(JP)

(72) Inventor: Funami, Tomoko
1-3-19, Inarimachi
Toyama-shi(JP)

(72) Inventor: Nagae, Shinji
23-4, Nakanoshin
Toyama-shi(JP)

(74) Representative: Bühling, Gerhard, Dipl.-Chem. et al,
Patentanwaltsbüro
Tiedtke-Bühling-Kinne-Grupe-Pellmann-Grams
Bavariaring 4
D-8000 München 2(DE)

(54) Diagnostic testing slide and diagnostic testing plate, and method of producing same.

(57) An immunochemical diagnostic testing slide made from an embossed (2) thermoplastic resin sheet (1) by subjecting said sheet to a denaturing treatment to provide the surface of the sheet with a hydrophilic nature, and an immunochemical diagnostic testing plate made by applying on the surface of the sheet with a hydrophilic nature an antigen- or antibody-sensitized water-insoluble carrier, followed by drying thereof.

FIG. 3

EP 0 111 042 A1

DIAGNOSTIC TESTING SLIDE AND DIAGNOSTIC

TESTING PLATE, AND METHOD OF PRODUCING SAME

This invention relates to a diagnostic testing slide and a diagnostic testing plate which can be conveniently used for the various immunochemical tests such as latex agglutination tests, and a method of producing such testing slide and plate.

With the advance of immunochemistry in recent years, the techniques have been established for detecting and measuring the quantities of the trace substances in body fluids by an immunochemical means making use of an antigen-antibody reaction conducted by sensitizing an antigen or antibody with the polystyrene latex particles. These immunochemical testing techniques made it possible to detect the trace substances such as peptide hormone, steroid hormone, protein, etc., contained in an in vivo fluid specimen such as blood, urine, etc., and are found especially useful in the fields of diagnostics and medical treatments. This immunochemical test is generally a method of detecting and measuring the quantities of the trace substances contained in a test fluid by observing the agglutination of the latex particles which is caused by an antigen-antibody reaction conducted between an antibody-sensitized polystyrene latex reagent and an antigen contained in the test fluid. Various methods have been proposed for sensitizing the antigen, including the methods using the erythrocytes, activated carbon, a metal oxide or the like as carrier. In said immunochemical test, a glass-made testing slide has been generally used for observing the agglutinated condition of the latex particles after mixing an antibody-sensitized latex reagent and a test fluid and then allowing the mixture to stand for a given period of time. However, such glass-made testing slide is usually expensive and also needs to be cleaned upon every run of test when a same slide is used repeatedly. Thus, use of such testing slide involved a risk of non-specific inhibition of the immuni-chemical reaction due to contamination by the residual detergent used for cleaning or other matters such as dust, oils, etc., or a scratch or other flaws on the glass surface, and this would affect the agglutination reaction of the latex particles in a qualitative test, resulting in a reduced agreement. Also, precise measurement would be often impaired in the qualitative tests, and thus utmost care was required for cleaning, preservation and treat-ment of the testing slide. With a view to eliminating these defects of the conventional glass made testing slides, a disposable testing slide made by coating the surface of a resin plate or paper with a coating material mixed with a nonionic surfactant has been proposed. This testing slide, however, still had a possibility of causing a non-specific agglutination reaction under the influence of the coating material or surfactant used. Also, in such slide, the dispersibility of the test material on the coated film would be deteriorated with time due to the volatilization of said surfactant. For these reasons, the heretofore proposed testing slides were unsuited for the diagnostic uses.

An object of this invention is to provide a diagnostic testing slide which is inexpensive, easy to handle in use for the immunochemical tests and stable in quality.

Another object of this invention is to provide a diagnostic testing plate which is formed on the surface of the testing slide by, for instance, spreading on said slide surface an antibody sensitized latex suspension of an amount necessary for one run of test and then drying the suspension and which is capable of detecting and measuring the quantities of the trace substances contained in a test fluid with ease and in a short period of time. The testing plate of this invention provides all the reagents necessary for an intended test on the slide, so that the desired immunochemical test results can be readily obtained by simply dropping a test fluid on the testing plate, and thus it is possible to accomplish an intended diagnostic test in a very short period of time with no need of performing any troublesome operations required in the conventional diagnostic tests of this type. This immunochemical diagnostic testing plate, which has thus strikingly simplified the testing operations, proves particularly convenient in use for a pregnancy test; any ordinary subject can freely perform the test and determine the results by herself with no need of taking any special examinations by a physician.

The base of the testing slide of this invention is formed from a thermoplastic resin such as ABS (acrylonitrile-butadiene-styrene) resin, PS (polystyrene), PVC (polyvinyl chloride), polyesters, PC (polycarbonate), PP (polypropylene), PE (polyethylene) or the like, with the surface thereof being properly treated to have a hydrophilic nature. Such thermoplastic resin sheet may be transparent, translucent or opaque and its color may be white, gray, black, etc. The surface of these thermoplastic resins usually shows an extremely high surface tension and is also hydrophobic, so that the untreated surface of such resins is poor in dispersibility of an aqueous fluid such as a latex solution, test fluid, etc., and hence such resins, if not properly treated, are unsited for use as the base of an immunochemical diagnostic testing slide. A testing slide usable for the immunochemical diagnostic examinations must have a surface which allows easy mixing of a latex suspension and a test fluid and also have an excellent dispersibility of the resultant mixture, and to meet such requirements, the testing slide needs to have a surface tension for water of desirably about 20 dyne/cm or less.

As a result of the extensive studies conducted by the present inventors for solving said problem, it was found that the surface tension of resin can be markedly reduced by subjecting the embossed resin surface either to (1) a flame treatment at a surface temperature which is 10° to 20°C higher than the thermal deformation temperature of the resin or to (2) a corona discharge treatment, and in consequence, both miscibility of a latex liquid and a test fluid and dispersibility of the resultant mixture on the resin surface are amazingly bettered. It was further found that the surface of the testing slide obtained according to the method of this invention is extremely stable physicochemically and can ideally lend itself to the formaion of a testing plate which can be obtained by applying a latex reagent solution on said slide and drying the solution. This invention was realized on the basis of these findings.

In the testing slide of this invention, the pressure or injection molded and embossed thermoplastic resin surface is further subjected to a flame or corona discharge treatment to impart a hydrophilic nature to the resin surface. The slide surface unevenness formed by embossing is preferably of a size within the range of 3.0 to 20 microns. The testing slide surface having the unevenness within the said size range porvides a good miscibility of a latex suspension and a test fluid and a good dispersibility of the resultant mixture on the slide surface to allow a very easy observation of the agglutination reaction of the latex particles. In case the size of the embossed surface unevenness is less than 3.0 microns, no satisfactory dispersibility of the resultant mixture (of a latex suspension and a test fluid)

on the slide surface is provided even if said surface is subjected to a flame or corona discharge treatment. On the other hand, if the size of said surface unevenness is greater than 20 microns, a difference is produced in optical density of latex on the resin surface due to a too large gap, hindering the observation of the agglutination reaction of the latex particles.

The embossing work on the testing slide can be accomplished by using a physical or mechanical treating means such as injection molding of a thermoplastic resin in a metallic embossing die or sand blasting on the resin plate.

Of the said testing slide surface denaturing treatments, the flame treatment is conducted by spouting the flames of propane gas against the resin surface to oxidize the polymer particles in the resin surface layer to thereby induce hydrophilicity on said testing slide surface. This treatment is perferably conducted at a surface temperature 10° to 20°C higher than the thermal deformation temperature (usually 90-90°C) of said resin for a period of 1 to 10 seconds. In the case of an ABS resin for instance, a 3-second treatment at 95° to 100°C can provide a testing slide having a surface with good dispersibility to the resultant mixture.

Another testing slide surface denaturing treatment usable in this invention, that is, the corona discharge treatment is performed in a usual way by applying a high-tension alternating current of a sine wave or logarithmic damped wave form with a crest value of 5 KV or above and in frequency range from commercial frequency to several hundred KHz. By this uniform corona discharge treatment on the slide surface, the terminal OH groups of the polymer particles in the resin surface layer are rearranged to induce hydrophilicity on the testing slide surface.

If desired, a circular or oval frame may be printed on the testing slide surface by using a hydrophobic paint to further facilitate the mixing of a test fluid and a latex suspension and the observation of agglutination of the latex particles.

BRIEF DESCRIPTION OF THE DRAWINGS

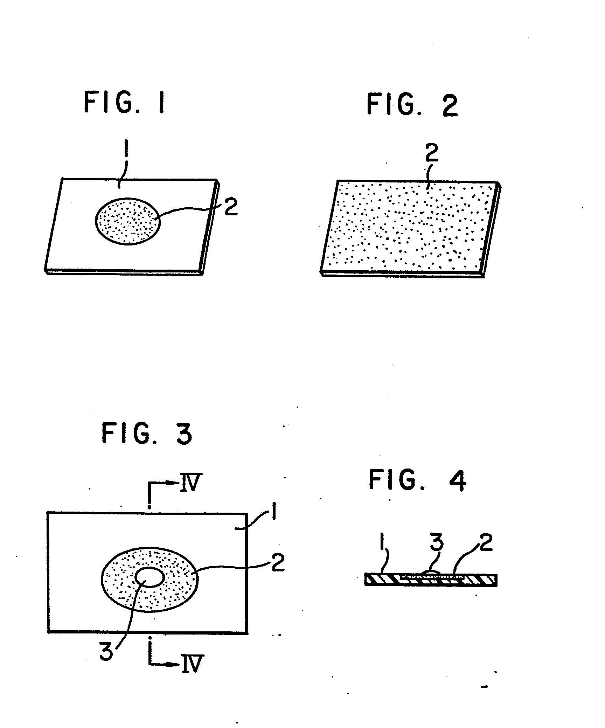

FIGS. 1 and 2 are the perspective views of a diagnostic testing slide according to this invention, FIG. 3 is a plane view of the upper surface of a diagnostic testing plate formed by spreading a reagent mixture on said testing slide, and FIG. 4 is a sectional view taken along the line 4-4 of FIG. 3.

The diagnostic testing slide and testing plate according to this invention will be described in greater detail hereinafter with reference to the accompanying drawings. It is to be understood however that these drawings merely illustrate some preferred embodiments of the invention and are not intended to limit the scope of th invention.

In the drawings, FIGS. 1 and 2 are the perspective views of a diagnostic testing slide according to this invention, FIG. 3 is a plane view of the upper surface of a diagnostic testing plate formed by spreading a latex reagent on the testing slide, and FIG. 4 is a sectional view of the testing slide as taken along the line 4-4 of FIG. 3. The whole or a part of the top resin surface 1 of the slide is embossed, as shown by 2, and this embossed portion or a part of the embossed portion of the resin surface is subjected to a denaturing treatment for providing the surface with a hydrophilic nature. An antigen- or antibody-sensitized latex reagent 3 is applied at the central part of the resin surface which has undergone said embossing and hydrophilicity-imparting treatments and then is dried.

When conducting an immunochemical reaction according to a conventional method, if the testing slide of this invention is used, such reaction can be accomplished by merely dropping a test fluid, a buffer solution and a latex reagent successively onto the testing slide surface. Also, since the testing slide of this invention is a disposable article, it can dispense with the troublesome washing work required for the conventional glass made testing slides. In the conventional immunochemical testing methods, for example, a method making use of an agglutination reaction of an antibody-sensitized latex; the test is usually conducted by using the dropping bottles or test tubes and, because of use of a latex suspension and a buffer solution, the testing operations are complicated: first a test fluid is dropped onto the testing slide, then a buffer solution is dopped and mixed with said test fluid, followed by further dropping and mixing of a latex suspension, and then the agglutination reaction of the latex particles is observed. Also, since each reagent for one shot is dropped by using a synthetic resin made pipette, the amounts of the respective reagents dropped might become inconstant to invite an inaccurate test result. Several proposals have been made for eliminating such defects of the conventional testing methods using said reagents. For instance, U.S. Patent 2,770,572 discloses a card used for deciding the blood type and U.S. Patent 3,074,853 teaches an antigen-antibody reaction on a plastic plate using activated carbon as carrier, while Japanese Patent Publication 46052/74 discloses an immunochemical testing slide containing the testing agents at the positions closely adjacent to each other. The testing slide of this invention can be best embodied in a form where the testing liquid chemicals are applied on the slide surface, and according to this invention, it is possible to produce and provide the testing plates containing a wide variety of immunochemical reagents at low cost. In practical use of the testing slide and testing plate of this invention, it is possible to use all known types of diagnostic reagents involved with the latex agglutination reaction using an antigen or an antibody, and by applying an accurately measured amount of a latex reagent

0111042

solution (for one run of test) on the slide surface and drying the solution, there can be obtained a diagnostic testing plate which is easy to use. Addition of saccharoid such as sucrose, mannitol, lactose, dextrane and the like in an amount of 0.2 to 10% in the latex reagent solution can further enhance the stability of the solution. Also, incorporation of other known techniques such as addition of normal rabbit serum, bovine serum albumin, surfactant or the like, adjustment of the pH and/or ionic concentration of the reaction system, etc., or a combination of some of these techniques, allows the advantageous practice of this invention.

The present invention will be described in further detail hereinbelow by way of the examples thereof. It is to be understood however that the invention can be emboided in various other forms within the scope of its purport and is not limited by the following examples.

Example 1

A metal mold having on one side a structure capable of transferring an emboss pattern with an approximately 6 micron-diameter unevenness was made, and an ABS resin was injection molded in this metal mold and the obtained resin plate was subjected to a flame treatment by ejecting the flames of propane gas to the embossed portion of the resin plate for a period of 3 seconds by maintaining the resin surface temperature at about 95° to 100°C to thereby obtain a diagnostic testing slide.

Example 2

A PS (polystyrene) resin plate was sand blasted with sand having an average particle size of 4.8 microns and the embossed portion of this resin plate was subjected to a corona discharge treatment by a 10 KV alternating current with a sinusoidal waveform of 600 KHz to obtain a diagnostic testing slide.

Now, the embodiments of the invention for the formation of a simple diagnostic testing plate by using the testing slide of Example 1 will be described in detail.

Example 3

A commercially available HCG (human chorionic gonadotropin) was refined to a specific activity of 10,000 IU/mg or higher by isoelectric electrophoresis, and by using this HCG as antigen, it was dissolved in a physiological saline solution to a concentration of 1 mg/ml, and 0.1 ml thereof was mixed with a complete Freund's adjuvant and the mixture was injected subcutaneously to the female house rabbits with body weights of 1.6 to 2.0 kg at a rate of one shot a week for the total 7 shots to immunize the rabbits. 8 weeks after the first injection, the whole blood of each test rabbit was collected from the carotid artery to obtain an antiserum. The thus obtained antiserum was refined by affinity chromatography to obtain an anti-HCG antibody. This antibody was diluted with a boric acid buffer or pH 8.2 to a concentration of 0.1% and 10 ml of this diluted solution was added to 10 ml of a 1% polystyrene latex suspension (particle size: 0.8 μm) and incubated at 40°C for one hour, followed by 30-minute centrifugation at 10,000 r.p.m. and additional washing with 10 ml of a boric acid buffer (pH 8.2). The resulting material was again suspended in 10 ml of a boric acid buffer (pH 8.2) containing 5% of sucrose, and the obtained latex reagent solution was accurately applied on the testing slides obtained in Example 1 at a rate of 30±2 μl per slide and then freeze-dried to reduce the water content of the reagent portion to less than 0.1%. The thus obtained testing plate exhibited an agglutination reaction in the presence of 1 IU/ml of HCG.

Example 4

2 Milligrams of human fibrinogen was dissolved in 1 ml of a physiological saline solution, and 0.1 ml of this mixed solution was mixed with a complete Freund's adjuvant and the mixture was injected subcutaneously to the female house rabbits with body weights of 1.6 to 2.0 kg at a rate of one shot a week for the total 5 shots. 6 weeks after the first shot, the whole blood of each test rabbit was collected from the carotid artery to obtain an antiserum, and this antiserum was refined by affinity chromatography to obtain an anti-human fibrinogen antibody. The thus obtained antibody was dissolved in a glycine buffer solution of pH8.6 to a concentration of 0.1% and 10 ml of this diluted solution was added to 10 ml of a 1% polystyrene latex suspension (particle size: 0.8 µ) and incubated at 40°C for one hour, followed by 30-minute centrifugation at 10,000 r.p.m., washing with 10 ml of a glycine buffer solution (pH 8.6) and re-suspension in 10 ml of a glycine buffer solution (pH 8.6) containing 1% of dextrane. The resultantly obtained latex reagent was applied on the testing slide obtained in Example 2 accurately at a rate of 25 ± 2 µl per slide and then freeze-dried to reduce the water content of the reagent portion to less than 0.1%.

The thus obtained testing plate demonstrated an agglutination reaction in the presence of 4-6 µg/ml of fibrinogen.

Other embodiments of this invention include a reagent kit for examining the functions of hypophysis and gonad by using an LH (luteinizing hormone) antibody or an LH antigen in an immunochemical test, and a reagent kit for determining estrogen by using FSH (follicle-stimulating hormone) and HMG (human menopausal gonadotropin).

0111042

WHAT IS CLAIMED IS:

1.      An immunochemical diagnostic testing slide made from an embossed thermoplastic resin sheet by subjecting said sheet to a denaturing treatment to provide the surface of the sheet with a hydrophilic nature.

2.      The immunochemical diagnostic testing slide according to Claim 1, wherein said denaturing treatment is a flame reatment.

3.      The immunochemical diagnostic testing slide according to Claim 1, wherein said denaturing treatment is a corona discharge treatment.

4.      An immunochemical diagnostic testing plate made by performing a denaturing treatment on the surface of an embossed thermoplastic resin sheet for providing said surface with a hydrophilic nature, and further applying on said surface an antigen- or antibody-sensitized water-insoluble carrier, followed by drying thereof.

5.      The immunochemical diagnostic testing plate according to Claim 4, wherein said denaturing treatment is a flame treatment.

6.      The immunochemical diagnostic testing plate according to Claim 4, wherein said denaturing treatment is a corona discharge treatment.

7.      The immunochemical diagnostic testing plate according to Claim 4, wherein said antibody is anti-human chorionic gonadotropin γ-globulin.

8.      The immunochemical diagnostic testing plate according to Claim 4, wherein said water-insoluble carrier is a polystyrene latex having a particle size of 0.1 to 1.0 micron.

9. A method of producing an immunochemical diagnostic testing slide characterized in that the surface of an embossed thermoplastic resin sheet is subjected to a denaturing treatment for providing said surface with a hydrophilic nature.

10. The method according to Claim 9, wherein said denaturing treatment is a flame treatment.

11. The method according to Claim 9, wherein said denaturing treatment is a corona discharge treatment.

12. A method of producing an immunochemical diagnostic testing plate which comprises performing a denaturing treatment on the surface of an embossed thermoplastic resin sheet for providing said surface with a hydrophilic nature, then further applying on said surface an antigen- or antibody-sensitized water-insoluble carrier and drying the same.

13. The method according to Claim 12, wherein said denaturing treatment is a flame treatment.

14. The method according to Claim 12, wherein said denaturing treatment is a corona discharge treatment.

15. The method according to Claim 12, wherein said antibody is anti-human chorionic gonadotropin $\gamma$-globulin.

16. The method according to Claim 12, wherein said water-insoluble carrier is a polystyrene latex having a particle size of 0.1 to 1.0 micron.

FIG. 1

FIG. 2

FIG. 3

FIG. 4

0111042

European Patent
Office

**EUROPEAN SEARCH REPORT**

Application number

EP 83 10 0070

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X,Y | WO-A-8 200 363 (R.A. HARTE et al.) <br> * Whole document * | 1,4,8, 12,16 | G 01 N 33/52 <br> G 01 N 33/54 |
| X | GB-A-2 087 074 (FUJI PHOTO FILM CO., LTD.) <br><br> * Whole document * | 1-3,5, 6,9-11 ,13,14 | |
| Y | GB-A-2 030 293 (SEIKAGAKU KOGYO CO., LTD.) <br> * Whole document * | 4,8,12 ,16 | |
| X,Y | US-A-3 770 383 (R.T. PRICE) <br><br> * Whole document * | 1,4,8, 9,12, 16 | TECHNICAL FIELDS SEARCHED (Int. Cl. ³) <br><br> G 01 N |

The present search report has been drawn up for all claims

| Place of search | Date of completion of the search | Examiner |
|---|---|---|
| THE HAGUE | 07-09-1983 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................

& : member of the same patent family, corresponding document

EPO Form 1503. 03.82